# EUROPEAN PATENT APPLICATION

(11) **EP 4 679 449 A1**
(43) Date of publication of application: **14.01.2026**
(21) Application number: 24186993.2
(22) Date of filing: 08.07.2024
(51) Int. Cl.: G16H 40/63, A61B 5/00, G16H 50/30

(54) **OBTAINING SENSOR DATA CORRESPONDING TO A TIME PERIOD**

(71) Applicant: Koninklijke Philips N.V., 5656 AG Eindhoven (NL)
(72) Inventor: HUIJBREGTS, Laurentia Johanna, Eindhoven (NL); VAN LIESHOUT, Ron Martinus Laurentius, Eindhoven (NL); BRANS, Harold Johannes Antonius, 5656AG Eindhoven (NL); NEUMANN, Rolf, Eindhoven (NL); BULUT, Murtaza, Eindhoven (NL)
(74) Representative: Philips Intellectual Property & Standards

(57) **Abstract**

A medical system comprising at least one sensor, configured to obtain data corresponding to a time period, a processor and a display. The processor is configured to determine a plurality of values of a parameter based on the data corresponding to the time period, wherein the plurality of values of a parameter are determined by at least two methods, rank the methods, and generate a prioritized method for the time period and at least one non-prioritized method for the time period based on the ranking. The display is configured to show each of a first window and a second window for a parameter, wherein in the first window the values of the parameter determined by the non-prioritized methods for the time period are not shown wherein in the second window at least one of the values of the parameter determined by the non-prioritized methods for the time period are shown, and wherein the processor further comprises a selector configured to select a prioritized and a non-prioritized window, out of the first and second window, wherein the selection adjusts a setting of the medical system

## Description

### TECHNICAL FIELD

The present invention relates to a medical system, in particular although not exclusively to a system and method for prioritizing the values of physiological parameters obtained by a processor and showing the values on a display.

### BACKGROUND

Medical systems such as patient monitoring systems are designed to measure, calculate and display a range of physiological parameters relating to a patient. These physiological parameters are utilized by healthcare professionals (HCPs), and other users of the system, to monitor the medical status of the corresponding patient and ensure the appropriate healthcare is provided. A hospital patient may be monitored with a range of sensors, and it is common for more than one sensor to measure the same physiological parameter. For example, the patient may wear an electrocardiogram (ECG) sensor to measure the electrical heart signal waveforms, a blood oxygen (SpO2) sensor to measure the oxygen saturation, and a HealthDot sensor to measure respiration rate, but they all can also measure heart rate. Due to intrinsic sensor differences and/or differences in the algorithms that derive the physiological parameter from the sensor signal, the values that are produced from different types of sensors can vary.

Existing patient monitoring systems currently show all values of the same physiological parameter that are measured. For example, the patient monitoring system may display the values of heart rate measured by an ECG sensor, SpOz sensor, and a HealthDot sensor. Furthermore, in current patient monitoring systems, the plurality of measurements of the same physiological parameter are displayed graphically in a similar way. Therefore, the HCP is provided with no indication as to which value of a physiological parameter is most important. For example, one value of a physiological parameter may be more trustworthy, reliable, or suitable for the present clinical setting.

Displaying a plurality of measurements for the same physiological parameter increases the cognitive workload of HCPs. For a HCP, it takes more time and more processing to read two values of a physiological parameter than it takes for a single value. Reading three values of the same physiological parameter takes more time and more processing than two values (and so on). Additionally, when more than one value is presented, the HCP is faced with an additional workload to decide which value should be relied on to make a clinical decision. However, in many cases, displaying a plurality of measurements of the same physiological parameter by a patient monitoring system provides no clinical advantage. Therefore, in existing systems, HCPs are burdened with surplus information which requires additional time, processing and decision making, for no benefit. The processing can lead to errors in the resulting healthcare, while the additional time required can cause delays in providing the healthcare. In addition, presenting surplus information also makes the system less user friendly, which is important when an untrained or non-medical user is relying on the system.

The present inventors have noted the above shortfalls and the importance of ensuring the HCP receives only the important information with a reduction in the number of irrelevant details. In most cases, it is sufficient for an HCP to only know a single value of a physiological parameter. This permits a faster response to the information, with a reduction in the number of decisions and mental processing needed from the user. However, in some cases, the HCP would like to inspect the values from multiple sensors. For example, the HCP may want to know if a value of a physiological parameter is being measured by a reliable sensor. In other situations, the HCP may want to see the differences between the multiple values of a physiological parameter, as this can reveal a physiological underlying cause (e.g., when there is a difference between heart rate from ECG and heart rate from photoplethysmography (PPG)). As a result, there is a need to show a limited amount of information most of the time and extended information when it is needed.

### SUMMARY

This Summary is provided to introduce a selection of concepts in a simplified form that are further described below in the Detailed Description. This Summary is not intended to identify key features or essential features of the claimed subject matter, nor is it intended to be used to limit the scope of the claimed subject matter.

In one aspect of the invention, there is provided a medical system comprising at least one sensor, a display and a processor, the processor further comprising a selector. The at least one sensor is configured to obtain data corresponding to a time period. The processor is configured to determine a plurality of values of a parameter based on the data corresponding to the time period, wherein the plurality of values of a parameter are determined by at least two methods, rank the methods, and generate a prioritized method and at least one non-prioritized method based on the ranking. The display is configured to show a first window and a second window, wherein in the first window, the values of the parameter determined by the non-prioritized methods for the time period are not shown, and in the second window the values of the parameter determined by the non-prioritized methods for the time period are shown. The display is configured to show the first window or the second window. The selector is configured to select a prioritized window and a non-prioritized window, out of the first or the second window, wherein the selection adjusts a setting of the medical system for the prioritized window and a non-prioritized window. The medical system settings refers to settings of the processor and/or display of the medical system.

Often a parameter of a patient is sensed by multiple sensors. In most cases, it is of no benefit to show all values from the different sensors to the HCP, as it does not improve their clinical decision making, while on the contrary, it will increase their cognitive workload, which may lead to errors or delay. Therefore, a medical system should show just the relevant information, which in most cases would be the values of only one sensor. By selecting the first window as the prioritized window, the HCP only receives the most important information and receives no irrelevant information.

While in most cases it is sufficient for a HCP to only know a single value of a physiological parameter, determined from data measured by one sensor, there are cases where showing multiple values is advantageous. In one example, this could be when the HCP is not sure if the sensor is still reliable. In another example, this could be in situations when the difference in the values of a parameter may reveal a physiological underlying cause (such as example when there is a difference the between heart rate from ECG and heart rate from PPG). Consequently, there is a need to show either limited information in the form of a single value of a parameter or all the information by displaying multiple values of a parameter.

In some embodiments, the value of a parameter for the time period may be shown on a graph. Optionally, the value of a parameter for the time period may be shown as a number. In some embodiments, the value of a parameter for the time period may be shown on a trendline. Optionally, the value of a parameter for the time period may be shown as a Visual Patient Avatar. In some embodiments, the value of a parameter for the time period may be shown as a numerical value. Optionally, the value of a parameter for the time period may be shown on a cardiopulmonary model. In some embodiments, the plurality of values of a parameter may be shown on separate graphs in the second window. In some embodiments, the plurality of values of a parameter may be shown on a single graph in the second window.

In some embodiments the methods may comprise determining a plurality of values of a parameter from one or more of different sensors and different algorithms.. For example, two methods of determining two values of heart rate may include determining the values from data obtained from two different sensors. Alternatively, two methods of determining two values of heart rate may include determining the values from data obtained from two different algorithms but using data from one sensor.

In some embodiments the number of the plurality of values of a parameter can exceed the number of sensors. Optionally, the number of the plurality of values may be the same as the number of sensors used to obtain data corresponding to a time period. It may be that at least two of the plurality of values of a parameter may be determined by the processor from the same sensor using at least two algorithms. It may be that at least two of the plurality of values of a parameter may be determined by the processor from the same sensor using at least two algorithms and the remaining plurality of values are each determined from data obtained from a unique sensor.

Determining a plurality of values of a parameter from one sensor is advantageous as it allows a more accurate value of a parameter to be generated. For example, SpO2 sensors may generate two values of a parameter by using two different calibration curves, a generic calibration curve and a skin-tone optimized calibration curve. In addition, a first algorithm for determining a parameter may be more suited for removing periodic motion artefacts, whereas a second algorithm for determining the same parameter may be more suited for removing non-periodic motion artefacts. Determining at least two of the plurality of values of a parameter from the same sensor using at least two algorithms and determining the remaining plurality of values from data obtained from a unique sensor is advantageous as it generates more values of a parameter, which gives greater choice to select the most optimal value in the first view, and provides greater amount of information for the HCP to determine an improved diagnosis in the second window. The same advantages exist for embodiments when the plurality of values are determined from a plurality of sensors, and, the number of the plurality of values is the same as the number of sensors used to obtain data corresponding to a time period. Moreover, a plurality of sensors provides improved safety if one sensor fails (e.g., the sensor provides inaccurate readings, or the sensor is not connected properly, or the sensor's battery dies) by ensuring a value of the parameter is still determined by a different sensor.

In some embodiments, the medical system settings may be adjusted to not show the non-prioritized window. In some embodiments, adjusting the setting of the medical system may comprise adjusting a setting of the display and/or processor. The display and/or processor settings may be adjusted to not show the non-prioritized window on the display area. By not showing the second window on the display area, the HCP is provided with only one value of a parameter when the first window is the prioritized window. This ensures the HCP is not supplied with any irrelevant or surplus information, and may lead to faster, more efficient and less mistake-prone decisions when caring for the patient. In some embodiments, the medical system settings comprise the brightness of the display, such that the brightness of the non-prioritized display is reduced relative to the prioritized display. In some embodiments, the medical system settings comprise the visibility of the display, such that the visibility of the non-prioritized display is reduced relative to the prioritized display.

In some embodiments the selector is further configured to automatically select the prioritized window and the non-prioritized window, out of the first or the second window, based on a first set of predetermined criteria. Automatic switching allows the most advantageous window to be shown to the HCP without further increasing the workload of the HCP.

In some embodiments the first set of predetermined criteria may include the difference between the plurality of values of a parameter. Optionally, if the difference between the plurality of values of the parameter is significant, and the accuracies of the plurality of values of the parameter are almost equal, then the second window may be selected as the prioritized window. This enables the HCP to make a judgment on which value of the parameter is most appropriate. Optionally, if the difference between the plurality of values of the parameter is insignificant the first window may be selected as the prioritized window. This ensures the HCP is not given irrelevant information.

A difference may be defined as significant or insignificant based on the percentage difference or an absolute value difference between the plurality of values of the parameter, which may depend on the type of parameter and/or its range. A difference may be defined as significant when one value of the parameter is in an alarm range and another value of the parameter is in a normal range. In some embodiments, the difference between the plurality of values of a parameter to automatically switch the prioritized window to the first window may not be the same as the difference between the plurality of values of a parameter to automatically switch to the prioritized window to the second window. For example, when a first determined value of MAP (mean arterial pressure) differs by more than 10 mmHg from a second determined value of MAP, the selector may select the second window as the prioritized window (if the second window was not already selected), but when the first determined value of MAP differs less than 4 mmHg from the second determined value of MAP, the selector may select the first window as the prioritized window (if the first window was not already shown). In some embodiments, the percentage or absolute difference may differ depending on how long a method of determining a value of a parameter has been prioritized. For example, if the value of heart rate determined from a first method has been prioritized for hours, then the difference between the plurality of values of heart rate required to switch the prioritized window to the second window view may have a less stringent threshold than if the value of heart rate determined from a first method was prioritized for only a short period of time. This provides the HCP with new information if when they have become familiar with the existing information. In addition, it provides the HCP with a chance to check the prioritized value is in-line with the values determined from the other sensor.

In some embodiments, the first set of predetermined criteria may include a value of the parameter passing a threshold value. Optionally, the first set of predetermined criteria may include the use of a new sensor. It is advantageous to show multiple values of a parameter when another sensor is attached to the patient as it allows a comparison to see if the values are similar; if they differ significantly, it might mean that the sensor (which might also measure other parameters) is not correctly attached, and the HCP can take action to improve it. In some embodiments, the first set of predetermined criteria may include the removal of a sensor, and the removal may be intentional or unintentional. Optionally, the first set of predetermined criteria may include the battery life of a sensor. In some embodiments, the first set of predetermined criteria may include the previous time each window has been shown. In some embodiments, the first set of predetermined criteria may include the previous time a method of determining a parameter has been the prioritized method. In some embodiments, the first set of predetermined criteria may include a reduction in the area of the display available to show a first window or a second window. It is advantageous to priorities the first window when the available screen size is reduced (e.g., because the caregiver is pulling up a menu on the screen) to make efficient use of the remaining screen size by providing the most relevant data. Optionally, the first set of predetermined criteria may include a patient's medical status. Optionally, the first set of predetermined criteria may include a patient's future medical status. It is advantageous for the patient's medical status to determine the prioritized window to enable the HCP to make improved decisions relevant to the clinical situation. For example, if the patient is in shock (e.g., hypovolemic/septic/cardiogenic shock) the first window should be prioritized, to enable a fast decision from the HCP. In other examples, the medical status of the patient may require more values of the parameter to be shown to enable to HCP to make an improved diagnosis, and so the second window may be prioritized. In some embodiments, the first set of predetermined criteria may include a HCP's preferences. Certain HCPs may have personal preferences to the information they are shown. For example, clinician A may always want to start a new shift with multiple values of a parameter, while clinician B may always want to start by seeing a single value. Optionally, the first set of predetermined criteria may include a workflow phase. For example, workflow phase may include a HCP's shift data. During certain times of a shift, it may be advantageous to show just one value of a parameter. For example, during handovers between HCPs additional values of a parameter would provide no benefit to the changing HCP and would cause distractions. Workflow phase may also include the availability of equipment, HCPs and other factors relating to the HCP workflow.

In some embodiments, the first set of predetermined criteria may be different for different parameters. Optionally, the first set of predetermined criteria may be different for different time periods. In some embodiments the selector may be further configured to select the prioritized window and the non-prioritized window, out of the first or the second window, based on a user interaction with the system. The user interaction may comprise input from a user via a touch screen, computer mouse, button, keypad, external device, gesture control or voice control or any other interaction of a user with the system.

In some embodiments the selector is further configured to select the prioritized window and the non-prioritized window, out of the first or the second window, based on a user interaction with the system and wherein the user interaction with the system overrides the automatic selection.

In some embodiments the selector is further configured to automatically select the prioritized window and the non-prioritized window, out of the first or the second window, based on previous user interactions.

In some embodiments, after generating an initial prioritized method and at least one non-prioritized method, the processor may only perform a further rank of the plurality of methods after a plurality of time periods. This is advantageous as it reduces computational power and makes the graph look less scattered. It is further advantageous for values of a parameter that require a minimum number of datapoints to determine the accuracy.

In some embodiments, after generating an initial prioritized method and at least one non-prioritized method, the processor may not re-rank the methods each time a plurality of values of a parameter are determined. This is advantageous as it reduces computational power and makes the graph look less scattered. It is further advantageous for values of a parameter that require a minimum number of datapoints to determine the accuracy.

In some embodiments, in the first window each value of the parameter shown may be uniquely stylized. Optionally in the second window each value of the parameter shown may be uniquely stylized. Optionally in the first and second window each value of the parameter shown may be uniquely stylized. This is advantageous as it shows when a new sensor is prioritized when the first view is shown. In some embodiments, the stylization may include size, color, darkness, or transparency of the value of the parameters. In some embodiments, the value of the parameter displayed in the first or second windows are labelled with text to indicate which method the value derives from.

In some embodiments, the ranking may be performed using the accuracy of the value. For example, the ranking may be performed in order of accuracy, wherein the method with the highest accuracy may receive the highest ranking. Optionally the ranking may be performed using the reliability of the value of the parameter. For example, the ranking may be performed in order of reliability, wherein the method with the highest reliability may receive the highest ranking. Optionally, the accuracy of the value of the parameter may be established using a predefined ranking of sensors. This is advantageous as it shows the HCP the most accurate value of a parameter in the first window. Optionally, heart rate derived from ECG is always ranked higher than heart rate from PPG, because ECG is expected to be more accurate, and on the other hand, heart rate from PPG is always ranked higher than that from accelerometery. Note that we focus on heart rate in this embodiment, but when focusing on another parameter, the ranking may be different. For example, in some embodiments, accelerometery is expected to be more accurate than PPG for respiration rate. In some embodiments, the HCP may adapt the default predefined ranking of sensors to produce a custom predefined ranking of sensors. In some embodiments, the accuracy of the value of the parameter may be established in combination with INOP (inoperative, i.e., technical alarms). For example, when there are no INOP alarms, the ranking follows the predefined ranking. Optionally, for heart rate the predefined ranking is 1. ECG, 2 PPG, 3 accelerometery. However, when a sensor is associated with an active INOP alarm, the expected accuracy drops. For example, if there is an INOP alarm "ECG leads off', then the ECG is expected to become least accurate and therefore the new ranking of expected accuracy is: 1 PPG, 2 accelerometery, 3 ECG. In some embodiments, the system itself derives the accuracy for each sensor from the raw sensor signals. Optionally, the ranking may be performed using a custom ranking.

In some embodiments, the ranking may be performed using the delay time between the patient's physiology and the determining of the value of the parameter. For example, when the value determined from a sensor lags behind the patient's real parameter value, the sensor may be given a lower ranking. This is advantageous because if there is a long delay, a change in physiology may be noticed late, and the patient may not receive timely care.

Optionally, the ranking may be performed using the delay time between the obtaining of data by a sensor and the determining of the value of the parameter by the processor. For example, when the measurement by a sensor takes a long time, or when the value determined from a sensor lags behind the real parameter value, the sensor is given a lower ranking. This is advantageous as it removes a source of delay from the system and ensure the most up-to-date values are shown to the HCP.

In some embodiments, the ranking may be performed using the computational cost needed to determine the value of the parameter. Optionally, the highest priority is given to the value of a parameter that is determined with the lowest computational power. This is advantageous as it prevents an overload of the processor. This is also advantageous in the case of a power failure, or when the system is powered by a battery. In some embodiments, the ranking is performed using computational cost when the sensor, display and/or processor is in an energy saving mode, for example when the sensor, display and/or processor is powered by a battery.

In some embodiments, the ranking may be performed using the common practice in the next care setting. For example, when the patient is going to be transferred to a lower acuity setting (such as from intensive care to a general ward), the sensor that is going to be used in that next care setting may be given a higher ranking. Optionally, the ranking may be performed using the favored sensor in the current, previous or future care setting. Optionally, the ranking may be performed using the battery level of the sensor. Optionally, if the at least one sensor is powered by a battery, those sensors get a lower ranking when their battery is low. In some embodiments, the ranking may be performed using the memory requirements of determining the value. In some embodiments, the ranking may be performed using predictive algorithms. Optionally, sensors for which hemodynamic stability index (HSI) has been trained may get a higher ranking when HSI has crossed a boundary for which hemodynamic instability is expected.

In some embodiments, the first and/or second window may also show which means of ranking is used. This provides the caregiver insight into how the system is operating, thereby removing the feeling of the system being a black box.

In some embodiments, if there have been at least two prioritized methods of determining the value of the parameter within a time window, wherein a time window comprises at least two time periods, then the processor is further configured to determine, using a second set of predetermined criteria and/or user input, which option has the higher importance out of showing a prioritized value of the parameter at each time period within the time window, wherein the prioritized value of the parameter at each time period within the time window refers to the value of the parameter determined from the prioritized method at each time period within the time window; and showing a single trend of the value of the parameter across the time window, wherein the single trend of the value of the parameter across the time window refers to the values of the parameter determined from a single method, which method was the prioritized method for at least one time period within the time window, and the first window shows the option with the higher importance.

In some embodiments, the second set of predetermined criteria may include one or more of the following factors: the parameter being determined, the patient's current medical status, the patient's historic medical status, a prediction of the patient's future medical status, the percentage of the time window that a method was the prioritized method, temporal characteristics of the value, the difference between the values of the parameter, a value of the parameter reaching a threshold value, the use of a new sensor, the removal of a sensor, the battery life of a sensor, the previous time each window has been shown, a reduction in the area of the display available to show a first or second window, a patient's medical status, a prediction of the patient's future medical status, a healthcare professional's preferences, and a health care professional's workflow phase. Optionally, the second set of predetermined criteria may also include one or more of the same factors as for the first set of predetermined criteria.

In some embodiments, in the second window, the ranking of methods is indicated. Optionally, the indication of the ranking of the methods may comprise using the vertical ordering of the graphs. In some embodiments, the indication of the ranking of the methods may comprise using multiple lines on a single line graph, with the ranking of the at least one non-prioritized method indicated by the formatting of the lines. The line formatting may include line thickness, transparency, color, darkness and/or pattern. For example, a thicker line may indicate a higher ranking than a thinner line, a more opaque line may indicate a higher ranking than a more transparent line, a darker color may indicate a higher ranking than a lighter color, or solid line may indicate a higher ranking than a dashed line. In some embodiments, the indication of the ranking of the methods may comprise using different datapoint markings on a scatter graph. The different data markings may comprise color, transparency, darkness, size and/or shape of the datapoint markings. In some embodiments, the ranking of the methods may be indicated by the size, color, transparency, or darkness of the number representing the value of the parameter. In some embodiments, the ranking of the methods may be indicated by an additional number next to the value of the parameter, the number representing the position in the rank of the value of the parameter. This is advantageous as the HCP can easily determine which data is most important.

In some embodiments, if the first window is selected to be the prioritized window, then the settings of the sensors used in a non-prioritized method may change. Optionally, if the first window is shown by the display, then the non-prioritized methods may be put into a sleep mode. In sleep mode, if a non-prioritized method uses a sensor, then the sensor may not obtain data, or if a non-prioritized method uses an algorithm, then the processor may no longer run that algorithm. Optionally, in sleep mode, the sensor may not transfer or store any further data. This may be advantageous in further extending the battery life of the hardware of the system, and in reducing the processing and computational costs of operating the system.

In some embodiments, the processor may be further configured to determine uncertainty data for the plurality of values of a parameter and show the uncertainty data in the first window. In some embodiments, the processor may be further configured to determine uncertainty data for the plurality of values of a parameter and show the uncertainty data in the second window. In some embodiments, the processor may be further configured to determine uncertainty data for the plurality of values of a parameter and show the uncertainty data in the first and second window. In one example, the uncertainty data is shown as error bars. In another example, the uncertainty data is shown as a range of values. This provides the HCP with additional data on the value of the parameter which will assist their interpretation of the true value of the parameter.

In one aspect of the invention, there is provided a method of displaying health data using a medical system, the medical system comprising at least one sensor, a display, and a processor, the processor further comprising a selector, the method comprising obtaining, by the at least one sensor, data corresponding to a time period, determining, by the processor, a plurality of values of a parameter based on the data corresponding to a time period, wherein the plurality of values of a parameter are determined by at least two methods; ranking, by the processor, the methods; generating, by the processor, a prioritized method and at least one non-prioritized method based on the ranking; showing, by the display, each of a first window or a second window, wherein in the first window the values of the parameter determined by the at least one non-prioritized method for the time period are not shown, and wherein in the second window the values of the parameter determined by at least one non-prioritized value of the parameter for the time period are shown, and selecting, by the selector, a prioritized window and a non-prioritized window, out of the first or the second window, wherein the selection adjusts a setting of the medical system.

### BRIEF DESCRIPTION OF THE DRAWINGS

Examples will now be described in detail with reference to the accompanying drawings in which:
Fig. 1 shows a medical system;
Fig. 2A illustrates a display wherein the first window is the prioritized window, and the settings of the medical system are adjusted to reduce the area of the display occupied by the non-prioritized window relative to the prioritized window;
Fig. 2B illustrates a display wherein the second window is the prioritized window, and the settings of the medical system are adjusted to reduce the area of the display occupied by the non-prioritized window relative to the prioritized window;
Fig. 3A illustrates a display wherein the first window is the prioritized window and the settings of the medical system are adjusted to reduce the area of the display occupied by the non-prioritized window relative to the prioritized window such that the non-prioritized display takes up none of the display area;
Fig. 3B illustrates a display wherein the second window is the prioritized window and the settings of the medical system are adjusted to reduce the area of the display occupied by the non-prioritized window relative to the prioritized window such that the non-prioritized display takes up none of the display area;
Fig. 4A illustrates a display wherein the second window is the prioritized window and the settings of the medical system are adjusted to reduce the area of the display occupied by the non-prioritized window relative to the prioritized window such that the non-prioritized display takes up none of the display area;
Fig. 4B illustrates a display wherein the first window is the prioritized window and the settings of the medical system are adjusted to reduce the area of the display occupied by the non-prioritized window relative to the prioritized window such that the non-prioritized display takes up none of the display area, and the single trend of values is more important than the prioritized values.
Fig. 4C illustrates a display wherein the first window is the prioritized window and the settings of the medical system are adjusted to reduce the area of the display occupied by the non-prioritized window relative to the prioritized window such that the non-prioritized display takes up none of the display area, and the prioritized values are more important than the single trend of values.
Fig. 5 shows a cardiopulmonary model; and
Fig. 6 shows a flow diagram of a method of displaying health data using a medical system.

The accompanying drawings illustrate various examples. The skilled person will appreciate that the illustrated element boundaries (e.g., boxes, groups of boxes, or other shapes) in the drawings represent one example of the boundaries. It may be that in some examples, one element may be designed as multiple elements or that multiple elements may be designed as one element. Common reference numerals are used throughout the Figures, where appropriate, to indicate similar features.

### DETAILED DESCRIPTION

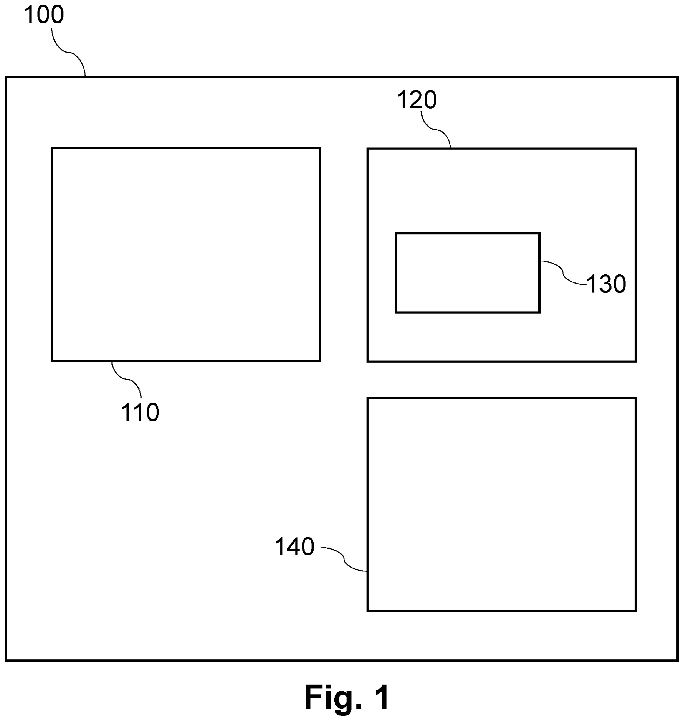

The following description is presented by way of example to enable a person skilled in the art to make and use the invention. The present invention is not limited to the embodiments described herein and various modifications to the disclosed embodiments will be apparent to those skilled in the art. Fig. 1 shows an embodiment of a medical system 100, comprising at least one sensor 110, a display 140 and a processor 120, the processor 120 further comprising a selector 130.

The sensor 110 generates data corresponding to a time period. The data may be associated with a physiological parameter of a patient. There may be various different types of sensor 110 obtaining data. There may be more than one sensor 110 obtaining data for the same physiological parameter. Sensors 110 may be able to obtain data for multiple physiological parameters. For example, a blood oxygen sensor may measure data corresponding to oxygen saturation and heart rate, and a breathing rate sensor may measure respiration rate and heart rate.

The processor 120 may receive the data from the sensor 110 and uses it to determine at least one value of a parameter. The sensor 110 may transmit the data to the processor 120 through wired or wireless means. The plurality of values of a parameter determined by the processor 120 may not all be the same, due to intrinsic differences in the type of data obtained by the sensor 110, the measurements performed by the sensor 110, the accuracy of the sensor 110, the algorithm used by the processor 120 and/or sensor 110, or any other reason.

A plurality of values of a parameter are determined from at least two methods. Methods may comprise determining data from different sensors 110 or from the same sensor 110 but using different algorithms. For example, two values of blood pressure may be determined from two methods, the first method comprising determining a first value using data from a first sensor, and the second method comprising determining a second value using data from a second sensor. Alternatively, the two values of blood pressure may be determined from two methods, the first method comprising determining a first value from a first algorithm using data from a first sensor, and the second method comprising determining a second value from a second algorithm using data from the first sensor.

The number of the plurality of values of a parameter can exceed the number of sensors 110. For example, three values of blood pressure may be determined by the processor 120 using data obtained one, two or three sensors 110. If the number of values of a parameter exceeds the number of sensors 110 from which data is obtained, then data from at least one sensor 110 may be used by the processor 120 to determine at least two values of the parameter. In such cases, the plurality of values may be determined from a plurality of algorithms. For example, if three values of respiration rate are determined by the processor 120 using data from one sensor 110, then three algorithms may be used to generate the three values of respiration rate. If three values of respiration rate are determined from two sensors 110, then one of the sensors 110 may generate one value of respiration rate, and one sensor 110 may generate two values of respiration rate using two algorithms.

As an example, an SpO2 sensor generates data on oxygen saturation by performing measurements on light signals. The processor 120 may determine the oxygen saturation using the data from the SpO2 sensor combined with a calibration curve that reflects the relationship between the oxygen saturation and the so-called "ratio of ratios", which is derived from the raw signal. Several calibration curves exist, and the system may contain several algorithms to determine the oxygen saturation, each algorithm using a different calibration curve. Providing multiple algorithms allows an accurate value of a parameter to be determined for a wider range of patients. In the above example, each algorithm may determine a different value of oxygen saturation, and the algorithm which produces the truest value may differ between patients depending on many factors (e.g., patient skin color).

Alternatively, a value of a parameter may be determined using data from a plurality of sensors 110. For example, a value of blood oxygen may be determined by the processor 120 using data from one, two, three or more sensors 110.

The processor 120 ranks of the methods used to determine the values of a parameter. By ranking the methods, each method may be given a number representing the order of the method in a list. The processor 120 may rank the method by determining a first ranked method and ranking all other methods as the joint second ranked method. Or the processor 120 may rank the methods by giving each method a unique ranking. Most importantly in the ranking is the determination of the top/first ranked method. For example, the system 100 may comprise three methods of determining heart rate, each method using data from three different sensors 110. The processor 120 may then rank the three methods to generate a first, second and third method. Or it may generate a first ranked method, and two joint second ranked methods.

The ranking may be performed by a variety of methods. Methods of ranking includes, but is not limited to: a predefined ranking of sensors 110, the accuracy of the value of the parameter, the reliability of the value of the parameter, the resolution of the sensor 110, the measurement frequency of the sensor 110, the delay time between the patient's physiology and determining the value of the parameter, the delay time between the obtaining of data by a sensor 110 and the determining of the value of the parameter by the processor 120, the computational cost needed to determine the value of the parameter, the battery level of the sensor 110, the patient's medical status, the patient's demographic, the memory requirements of the value of the parameter, the favored sensor 110 in the current care setting, or predictive algorithms. The ranking may be performed based on any combination of the methods used in the above list. For example, the ranking may be performed based on the accuracy of the value of the parameter and the delay time of the sensor 110.

The ranking may be performed based on the accuracy of the sensor 110 used in the method. For example, the sensors 110 may previously be ranked according to the known accuracy of the sensors 110. This could be based on the accuracy or precision quoted by the manufacturer for the sensor 110. For example, one sensor 110 may be configured to measure heart rate to within 1 beat per minute (BPM), whereas a second sensor 110 may be configured to measure heart rate to 5 BPM. The more accurate sensor 110 would rank higher in this method of ranking. Alternatively, it may be based on the experience of an HCP on the accuracy of the sensors 110. For example, based on experience with the sensors 110, the value of body temperature from a first sensor 110 may be believed by an HCP to be more accurate than the value from a second sensor 110, and the value of body temperature from the first sensor 110 will be ranked higher.

The ranking of accuracy may further comprise data according to associated INOP alarms. For example, if an INOP alarm is active for a sensor 110, its accuracy level drops. In this case, with no active INOP alarms, the ranking may be (based on accuracy), in descending order, the value determined from a first sensor, a second sensor, and a third sensor. If an INOP alarm is then active for first sensor 110, its expected accuracy may drop such that it becomes the second or third ranked sensor 110. In another example, the value of heart rate determined from data measured by an ECG sensor may be ranked top. However, if an INOP alarm of "ECG leads off' is active for the ECG sensor, its predetermined accuracy may drop, such that the value is ranked lower.

The ranking may be performed using the delay time between the patient's physiology and the determining of the value of the parameter. For example, there may be a delay between a patient's heart rate being a certain value and the system determining said value. As a result of this delay, the value determined may no longer be the actual value of the patient's physiology. In some embodiments, this delay may be due to the method of determining that is used. For example, there may be a delay for a change in concentration of a biomarker in the patient's blood to show up in the biomarker concentration measured in sweat, and this delay results from the biomarker first needing be transported from the blood to the sweat, to the outside of the skin, and then to the sensor. In some examples, sensors only perform measurements intermittently, and there may be a delay time between two consecutive measurements. When the value determined from a sensor lags behind the patient's real parameter value, the sensor may be given a lower ranking. This is advantageous because if there is a long delay, a change in physiology may be noticed late and the patient may not receive timely care.

The ranking can be performed based on the delay time between the obtaining of data by a sensor 110 and the determining of the value of the parameter by the processor 120. For example, if a long time is required to determine the value of the parameter, said value may be given a lower ranking. The delay time between obtaining the data and determining a value of a parameter may be determined for each value of the parameter and used to generate a ranking. Alternatively, the ranking may be based on another method (for example, accuracy of the value of the parameter) but additionally utilizing the delay time to further rank the values. For example, if delay time is minimal for all values of a parameter, then the ranking follows the accuracy of the values. However, if one sensor 110 has a significant delay time, then its value of a parameter may be given a lower ranking relative to the ranking based exclusively on accuracy. A delay time may be determined to be significant relative to other delay times, or by comparison to predefined delay times. For example, a delay time may be significant if it exceeds one second, or five seconds, or ten seconds, or a minute. Alternatively, a delay time may be determined to be significant, if, in the time taken to determine the value of the parameter, the true value of the parameter has changed, such that the value of the parameter determined is no longer representative of the real value.

The ranking may be performed based on the computational cost needed to determine the value of the parameter. For example, the value of a parameter that is determined with the lowest computational cost may be given the highest ranking. The computational cost may refer to the processing power needed to obtain the data and/or determine the value of the parameter. The computational cost may refer to the data storage and/or communication cost, and the ranking performed based on the capacity of data communication between the sensor 110 and processor 120 and/or data buffer capacity on the system. For example, a value of a parameter that uses the lowest amount of data (bytes) may be given the highest priority. The value of the parameter determined with the highest or lowest resolution and/or measurement frequency may be given the highest priority.

The ranking may be performed based on the patient's medical status. For example, when the patient's perfusion index is low, the ranking of the methods to determine SpO₂ may change. The patient's medical status may include their current or past treatment. For example, when the patient is taking beta-blockers, which can reduce perfusion of the extremities, an alternate sensor which is not on the extremities, for example a nasal SpO2 sensor, could be given a higher position in the ranking than a finger SpO2 sensor.

The ranking may be performed based on the battery level of the sensor 110 used to obtain the value of the parameter. For example, the value of the parameter obtained from the sensor 110 with the highest battery level may be given the highest ranking. Alternatively, the ranking may be based on another method, for example, accuracy of the value of the parameter, but additionally utilizing the battery level of the sensor 110 to further rank the values. For example, if battery level is sufficient for all values of a parameter, then the ranking follows the accuracy. However, if one sensor 110 has a low battery level, then its value of a parameter may be given a lower ranking relative to the ranking based exclusively on accuracy. Alternatively, if one sensor 110 has a high battery level, then its value of a parameter may be given a higher ranking relative to the ranking based exclusively on accuracy. A battery level may be determined to be high or low based on the relative battery levels of all the sensor 110. A battery level may be determined to be high if the battery level exceeds a set percentage of total battery capacity, for example 50%, 60%, 70%, 80% or 90% of total battery capacity. A battery level may be determined to be low if the battery level is below a set percentage of total battery capacity for example 50%, 40%, 30%, 20% or 10% of total battery capacity.

The ranking may be performed based on the favored and/or available sensors 110 in the previous, current, or future care setting. For example, certain hospital wards may prefer to see values of a parameter obtained from a specific sensor 110 or certain wards may only have access to certain sensors 110, and thus those derived values may be given the highest priority. The ranking may be performed based on the favored sensor 110 or on the available sensors 110 in the next care setting. A patient may be moved to a different care setting, for example from one hospital ward to another, and the favored sensor 110 may be different on each ward. The processor 120 may give the highest rank to the value determined from a sensor 110 which is favored on the next ward.

The ranking may be performed based on predictive algorithms. For example, when HSI has crossed a boundary for which hemodynamic instability is expected, sensors for which HSI has been trained may be given the highest ranking.

The ranking may be based on the length of time the current highest rank sensor has been the highest ranked sensor. For example, if a method of determining a value has been the top ranked method for a long time, the system may lower the ranking of this sensor. This increases the likelihood of providing the HCP with new information when they have become familiar with the existing information. In addition, by making it more likely to rank an alternative value of a parameter as the highest ranked method, the probability of an erroneous value remaining the highest ranked is reduced. This improves the reliability of the system. Alternatively, if a method of determining a value has been the top ranked method for a long time, the system may increase the ranking of this sensor. This is advantageous as the HCP has become familiar with a single method of determining the value of a parameter, and this familiarity can benefit medical decision making. For example, it allows the HCP to understand trends in the parameter, or enables them to have increased trust in the method of determining the parameter.

Once the processor 120 has ranked the values of the parameter, the processor 120 generates a prioritized method and at least one non-prioritized method based on the ranking. It follows that the values of the parameter determined from the prioritized method are also prioritized, and the values of the parameter determined from the non-prioritized method are also not prioritized. The prioritized value refers to the value determined from the prioritized method at a time period and the non-prioritized value refers to the value determined from the non-prioritized method at a time period. The prioritized method may be the top ranked method, and the other ranked method may be the non-prioritized method. For example, if the processor 120 has ranked three methods of determining values of body temperature to generate a first, second and third ranked method, the first ranked method may be the prioritized method and the second and third ranked method may be the non-prioritized methods. If the processor 120 has ranked three methods of determining values of body temperature to generate a first, ranked method and two joint second ranked methods, the first ranked method may be the prioritized method and the two joint second ranked method may be the non-prioritized methods.

After generating an initial prioritized method and at least one non-prioritized method, the processor 120 may only perform a further rank of the at least two methods after a plurality of time periods. Alternatively, after generating an initial prioritized method and at least one non-prioritized method, the processor 120 may not re-rank the at least two methods each time a plurality of values of a parameter are determined. Optionally, after performing a ranking, the processor 120 may not re-rank the methods for the duration of a time window, where a time window corresponds to at least two time periods.

Once values of a parameter are determined based on data corresponding to a time period, and the methods of determining the values are ranked to generate a prioritized and at least one non-prioritized method, the processor 120 may either only re-rank after a plurality of time periods or not re-rank the methods at the next time period. The processor 120 may continue to determine at least one value of the parameter at the next time period, but the ranking of the methods remains the same as the last time period. As a result, the prioritized and non-prioritized methods at the second time period are the same methods as the for first time period. As an example, at the first time period, the value of heart rate obtained from an ECG sensor may be the highest-ranking and prioritized method of determining a value of heart rate, and the value obtained from a blood oxygen sensor was given the second ranking and was a non-prioritized value. At a second time period, new data is obtained by the sensor 110, and new values of heart rate are determined, however, the processor 120 does not re-rank the methods of determining heart rate, and the new value of heart rate determined by the ECG sensor remains the highest ranked and the prioritized value. This may continue for a plurality of time periods, until the processor 120 is configured to re-rank the methods. For example, the processor 120 may be configured to re-rank after a set number of time periods, or the processor 120 may re-rank after a set time. Alternatively, the processor 120 may only re-rank based on another stimulus, including but not limited to the value of the parameter passing a threshold, the value of another parameter passing a threshold, the patient moving into a different care setting, the activation of an INOP alarm, the number of sensors 110 changing, the appearance of another alarm, the battery of a sensor 110 passing a threshold.

The display 140 is configured to show each of a first window and a second window for a parameter. Each is intended to mean that the display 140 is configured to show a first window and a second window, but it may be that the display only shows one of the first window or second window for any one parameter for any one time period. In cases where the display shows two different parameters, such as heart rate and blood pressure, the display may simultaneously show a first window for a first parameter and a first window for a second parameter, or a first window for a first parameter and a second window for a second parameter, or a second window for a first parameter and a first window for a second parameter, or a second window for a first parameter and a second window for a second parameter. However, in some embodiments, the display may not show a first window for a first parameter at the same time as showing a second window for a second parameter.

In the first window, values of the parameter determined by the non-prioritized methods for the time period are not shown. In the first window, the only value of the parameter for the time period shown is the value determined by the prioritized method, and no value determined by other methods is shown. For example, if the prioritized method of determining body temperature has a value for a time period of 37.8°C, then in the first window, 37.8°C is the only value of body temperature shown for the time period.

In the second window the values of the parameter determined by the non-prioritized methods for the time period are shown. The non-prioritized methods may include all of the methods of the parameter. In other words, all of the values of the parameter are shown. The prioritized value of the parameter may also be shown in the second view. This provides the HCP with all information that is obtained regarding a physiological parameter.

The display 140 may show a first or a second window for a plurality of parameters. For example, the display 140 may be configured to show each of a first window and a second window for parameters including but not limited to heart rate, blood pressure, respiration rate (RR), body temperature, blood oxygen (SpO2), global end-diastolic volume (GEDVI), systemic vascular resistance index (SVRI), continuous cardiac index (CCI), stroke volume index (SVI), extravascular lung water index (ELWI), central venous pressure (CVP), systolic blood pressure (SBP), mean arterial pressure (MAP). The display 140 may show the first window simultaneously for all parameters. The display 140 may show the second window simultaneously for all parameters. The display 140 may show any combination of the first window and second window simultaneously for all parameters. For example, for one time period the display 140 may show only the prioritized value of heart rate and only the prioritized value of blood pressure. In another time period, the display 140 may show only the prioritized value of heart rate but all values of blood pressure. In another time period, the display 140 may show all values of heart rate but only the prioritized value of blood pressure. In another time period, the display 140 may show all values of heart rate and all values of blood pressure.

In the first and/or second window, the values of the parameter may be shown on a graph, as a numerical value, as a waveform, as a Visual Patient Avatar or as a cardiopulmonary model. The graph may be a trendline, Frank-Starling curve, waveform, or any other graph. The graph may be depicted as a line graph, scatter graph, bar graph, or any other suitable visualization. If the value of a parameter is shown on a graph, values of the parameter for previous time periods may also be shown. For a Visual Patient Avatar, the value may be shown in a first window in a variety of ways, including but not limited to, displaying a number, color or other graphical representation of the value using an avatar. The second window may also be shown in the Visual Patient Avatar in various ways, including but not limited to the avatar switching back and forth between various values, for example by flickering between violet and light brown to reflect that one sensor is in the low SpO2 range, while the other is in the normal SpO2 range. Optionally, the time displaying the highest prioritized value is longer than that of a lower ranked value (e.g., flickering with alternating 400 milliseconds in violet and 200 milliseconds in light brown). Optionally, when the value of the parameter is shown as a number, the color of the number may be used to represent if the value is within or outside of a normal or expected range.

In the second window, the plurality of values of a parameter may be shown on a single graph with a single set of axes, or on a single graph with multiple axes, or on a plurality of graphs. For example, the plurality of values of a parameter may be shown on a single graph as multiple lines, or scatter points, or any other representation. Alternatively, each value of a parameter may have its own graph or axes.

The value of one parameter may be shown as a graph, whereas the value for another parameter may be shown as a numerical value, as a Visual Patient Avatar, as a waveform, or as a cardiopulmonary model. For example, in the first and second view, heart rate may be shown as a graph whereas in the first and second window, the value of blood pressure is shown as a numerical value.

The value of a parameter may be shown in a first window and second window in the same form, that is both are shown on a graph, as a numerical value, as a Visual Patient Avatar or as a cardiopulmonary model. Alternatively, the value of a parameter may be shown in a first view on a graph, as a numerical value, as a Visual Patient Avatar, as a waveform, or as a cardiopulmonary model, whereas the value of the same parameter in a second view may be shown in a different form. For example, the first window may show the prioritized value of heart rate as a numerical value, but the second window may show all the values of heart rate on a graph.

In the first window the method used to rank the methods of determining the values may also be shown. In the second window the method used to rank the methods of determining the values may be shown. The display 140 may show the method of ranking using text and/or a graphical icon. By way of example, the display 140 may show the method of ranking by showing a message stating, "METHOD OF RANKING: SENSOR ACCURACY". The method of ranking may be shown permanently, periodically, or when prompted by a user. This provides the HCP with details on the method the system 100 is using, enabling them to make an informed decision whether to use the information shown in the first window, change the method of ranking, or change the display 140 to show a second window.

In the second window, the display 140 may also indicate the ranking of the methods of determining the values of the parameter. The ranking may be indicated in any way capable of distinguishing the methods. For example, if the methods of determining the values of the parameter are shown on a graph, the indication of the ranking may comprise using multiple lines on a single line graph, with the ranking of the at least one non-prioritized method is indicated by the formatting of the lines. The line formatting may include but is not limited to line thickness, color, transparency, darkness and/or pattern. For example, a thicker line may indicate a higher ranking than a thinner line, a more opaque line may indicate a higher ranking than a more transparent line, a darker color may indicate a higher ranking than a lighter color, or solid line may indicate a higher ranking than a dashed line. In some embodiments, the indication of the ranking may comprise using different datapoint markings on a scatter graph. The different datapoint markings may include, but is not limited to, data marking color, style, transparency, darkness, size and/or shape. Optionally, if the plurality of values are shown on a plurality of graphs, the indication of the ranking may comprise using the vertical ordering of the graphs. If the values of the parameter are shown as numbers, the ranking may be indicated by the size, color, transparency, or darkness of the number or any other way of indicating the ranking. The ranking may be indicated by an additional number near the value of the parameter, the number representing the position of the value in the ranking. For example, the highest ranked value of heart rate may have a number one near it, and the second ranked value may have a number two near it and so on. The number of the ranking may be formatted such that it can easily be distinguished from the number of the value of the parameter, for example by appearing in superscript.

The display 140 may comprise a plurality of displays. For example, the system may have a plurality of displays 140. There may be a primary display, which remains local with the patient, and a plurality of secondary displays. For example, an external mobile device may act as the display, allowing an HCP to view the display remotely to the patient. The number of displays may not be fixed.

The processor 120 may be further configured to determine uncertainty data for the methods of determining the plurality of values of a parameter. The uncertainty data may then be shown by the display 140 in the first and/or second window. The processor 120 may calculate the uncertainty data based on the data obtained by the sensor 110. For example, the uncertainty data may be the range or standard deviation or any other statistical measurement on the data obtained by the sensor 110. Alternatively, the uncertainty data may be based on the manufacturers stated uncertainty data. For example, the manufacturer may quote the accuracy of a body temperature as +/- 0.5°C. Alternatively, the uncertainty data may be based on a user's determination of the accuracy of the sensor 110. The display 140 may then show the uncertainty data for the values of a parameter in the first and/or second window. The uncertainty data may be shown, for example, as error bars, shaded regions of a graph, +/- values, or as a range of values.

In the second window, the values of a parameter may be represented as distinct values/lines/plots. Alternatively, the values of a parameter may be converted into statistical data. For example, in the second window, the values of heart rate may be shown as a weighted average with error bars. Optionally, in this weighted average, the data from the sensors 110 with a high ranking may weigh more than the data from sensors 110 with a low ranking. The error bars may be determined from the spread in the data from the various sensors 110 or from any other appropriate statistical measurement.

The processor 120 further comprises a selector 130 configured to select a prioritized window and a non-prioritized window, out of the first or the second window. The selection then adjusts a setting of the medical system for the prioritized window and a non-prioritized window.

The medical system settings may be adjusted in various ways to enable the prioritized window to be more dominant than the non-prioritized window. For example, the system may be adjusted to allow the prioritized window may take up a greater proportion of the display area relative to the non-prioritized window. The prioritized window may take up over 60%, 70, 80, 90 or 100% of the display area occupied by the first and second window. Optionally, the non-prioritized window may not take up any of the display area. Alternatively, the non-prioritized window may be more transparent than the prioritized window, and/or in lighter or less visible colors than the prioritized window. Optionally, the brightness of the medical system settings may be adjusted to make the prioritized display more visible relative to the non-prioritized display. The prioritized and non-prioritized windows may occupy the same region of the display, or overlapping regions of the display, or different regions of the display. The medical system settings may be adjusted to blur the non-prioritized window or conceal it entirely. By adjusting the medical system settings, the HCP is provided with the most relevant information, and the amount of surplus information is reduced. The medical system settings may be adjusted in any other way that reduces the visibility of the non-prioritized display relative to the visibility of the prioritized display. Adjusting the medical system settings may require adjusting the settings of the display and/or processor to achieve the above effects.

The selector 130 may be further configured to automatically select the prioritized window and the non-prioritized window, out of the first or the second window, based on a first set of predetermined criteria. The first set of predetermined criteria may include, but is not limited to, one or more of the following factors: a value of the parameter reaching a threshold value, the use of a new sensor 110, the removal (both intentionally and unintentionally) of a sensor 110, the battery life of a sensor 110, the previous time each window has been shown, a reduction in the area of the display 140 available, a patient's medical status, a patient's predicted future medical status, a healthcare professionals preferences or a healthcare professional's shift data and the difference between the values of a parameter. The first set of predetermined criteria may include more than one of the abovementioned criteria in combination, and the predetermined criteria may differ between time periods.

The first set of predetermined criteria may include the difference between the values of a parameter. The difference may be the mathematical difference between the values of a parameter. For example, if a first value of heart rate is 80 BPM, and a second value is 70 BPM, the difference will be 10 BPM. If the difference between the plurality of values of the parameter is insignificant the first window may be selected as the prioritized window. If the difference between the plurality of values of the parameter is significant, and the accuracies of the plurality of values of the parameter are almost equal, then the second window may be selected as the prioritized window. A difference may be defined as significant or insignificant based on the percentage difference or the absolute difference between the plurality of values of the parameter. For example, a difference may be deemed significant if it is greater than 10%, greater than 20%, greater than 30% or greater than 40% of the value of the parameter. A difference in a parameter, for example body temperature, may be significant if the difference compared to other values of body temperature is greater than 0.5°C. A percentage or absolute difference may be deemed significant for one parameter, whereas the same difference for another parameter may be deemed as insignificant. For example, when a first determined value of MAP differs by more than 10 mmHg from a second determined value of MAP, the selector may select the second window as the prioritized window (if the second window was not already selected), but when the first determined value of MAP differs less than 4 mmHg from the second determined value of MAP, the selector may select the first window as the prioritized window (if the first window was not already selected).

The first set of predetermined criteria may include a value of the parameter passing a threshold value. For example, a parameter may have a range of values deemed as medically safe, and when the value of the parameter passes outside this range, it is said to be within an alarm range. If at least one value of a parameter is in an alarm range, and at least one value of the parameter in in a normal range, then the second window view may be selected as the prioritized window.

The first set of predetermined criteria may include the use of a new sensor 110. For example, if a sensor 110 is attached to a patient that measures a parameter that is already being measured by one or more other sensors 110, the second window may be selected as the prioritized window. This allows the HCP to see all the values of the parameter, and if they are judged to differ significantly it might mean that the newly added sensor 110 is not attached correctly, allowing the HCP to reattach the sensor 110.

The first set of predetermined criteria may include the length of time each window has been selected as the prioritized window. For example, if a value of a parameter has been shown in a first window for a significant length of time, the selector 130 may be more likely to select the second window as the prioritized window. If a value of a parameter has not been shown for a significant length of time, the selector 130 may be more likely to select the first window as the prioritized window.

The first set of predetermined criteria may include the previous time a method of determining a parameter has been the prioritized method. For example, if the value of heart rate determined from a first method has been prioritized for hours, and the first window has been prioritized for a similar length of time, then the selector may be more likely to select a second window. This provides the HCP with new information when they have become familiar with the existing information. In addition, by making it more likely to show an alternative value of a parameter when the first window has been showing the value of a parameter determined from the same method for a long time, the possibility of showing an erroneously prioritized value is reduced. This improves the reliability of the system.

The first set of predetermined criteria may include a reduction in the area of the display 140 available. When the available screen size of the display 140 is reduced (e.g., because the caregiver is pulling up a menu on the display) the selector 130 may select the first window as the prioritized window. This is because the first window displays fewer values of a parameter and requires less space of the display 140.

The first set of predetermined criteria may include a patient's medical status. For example, if the patient status is determined to be medically critical (e.g., the patient has entered a state of hypovolemic/septic/cardiogenic shock) then the first window may be selected as the prioritized window. This provides the HCP with a quick overview of the vitals (without getting distracted by details) allowing them to make fast decisions on interventions.

The first set of predetermined criteria may include a HCP's preferences. The HCP may have a preferred window that they want shown by the display 140 for most of the time. The predetermined criteria may also include a healthcare professional's shift data. For example, the phase in the workflow of the HCP may determine the view, such as during handover (e.g., from a HCP in the day shift to a HCP in the night shift). During handover, the selector 130 may select the first window as the prioritized window, because only one value of a parameter is needed to provide a summary of the overall past and present patient status, allowing only the important details will be discussed by the HCPs without additional details which may cause unnecessary distractions.

The selector 130 may be further configured to select the prioritized window and the non-prioritized window, out of the first or the second window, based on a user input, user interaction or a manual input. A non-exhaustive list of examples includes but is not limited to input/interaction from a user via a touch screen, external device, computer mouse, button, gesture control, keypad or voice control. The input may also come via a secondary device such as a mobile device or tablet. The manual input may also be further configured to override the automatic selection of the window to be shown by the display 140. For example, at a time period the selector 130 may be configured to select the first window as the prioritized window, based on the predetermined criteria, however, an HCP can override this selection using manual input, and select the second window to be the prioritized window. The manual input may also be configured to only override the automatic selection for a fixed period of time. For example, if the manual input has selected the second window to be the prioritized window, without further manual input the system may select the second window for a fixed time period as the prioritized window (for example, 1 minute) before reverting back to the automatic selection made by the predetermined criteria.

In some embodiments the selector is further configured to automatically select the prioritized window and the non-prioritized window, out of the first or the second window, based on previous user interactions. If the system receives manual input from the user, it may take these interactions into account when automatically selecting the window. The selector may learn to spot patterns for when a HCP interacts with the system, and based on this learning, the selector may automatically select a window. For example, if an HCP often interacts with the system to select the second window as the prioritized window at a certain time in their shift or in response to a specific status of the patient, then the system may learn this behavior and automatically select the desired system at the point the manual interaction is expected, or the patient's status is similar. In another example, the system may learn how a HCP's role (nurse, intensivist, anaesthesiologist, etc.) impacts their interaction with the system, and automatically select the window based on this learning. It may be that when the HCP is a nurse, the first window is often selected as the prioritized window, as such, the system may learn this preference and account for it in its automatic switching.

If the selector 130 selects the first window as the prioritized window, then the ranking of the methods may only determine a first ranked method and rank all other values as joint second. In other words, if the first view is shown, it is important that the best method has been determined, but it may not be necessary to rank the remaining values as 2nd, 3rd, etc. if the second window is selected as the prioritized window, the further ranking of the joint second ranked methods may be performed (i.e. also the non-top ranked values should be ordered, as 2nd, 3rd, etc.).

The system 100 may be further configured to put a sensor 110 into a sleep mode. Sensors 110 may be put into sleep mode when their data is not used to generate a value of the parameter shown by the prioritized window during that time period. For example, for time periods when the first window is the prioritized window and the data corresponding to a time period obtained by the sensor 110 is not used in the determination of the prioritized value of the parameter, which sensors 110 will be put into sleep mode. When in sleep mode, the sensors 110 may not obtain data, store data, or transfer data to the processor 120 for a time period. The processor 120 may not determine a value of a parameter based on the data from a sensor 110 in sleep mode. This allows the system 100 to save battery, communication bandwidth, computing power, and/or storing capacity. If the selector 130 selects the second view as the prioritized window, every value of a parameter may be displayed, and so no sensor 110 will be in sleep mode. There may be a toggle on/off button to activate/deactivate measurements from deprioritized sensors 110.

During a time period, a method of determining a value of a parameter may determine any number of values of a parameter. For example, during a time period, a heart rate sensor may determine 0, 1, 2, 3 etc. values of heart rate. It may be possible that a first method of determining a parameter has a different sampling frequency than another method of determining the same parameter. For example, a first method of determining heart rate may determine 5 values for a time period, whereas a second method may determine 1 value for the same time period, whereas a second method may determine 0 values for the same time period.

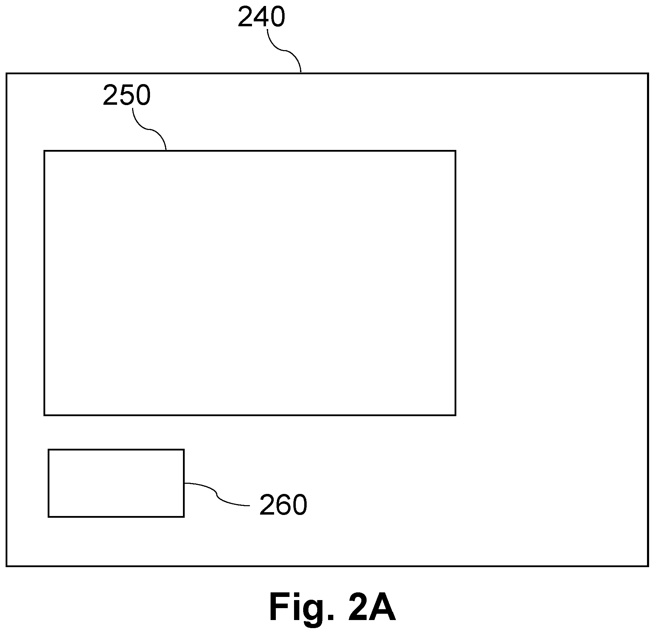

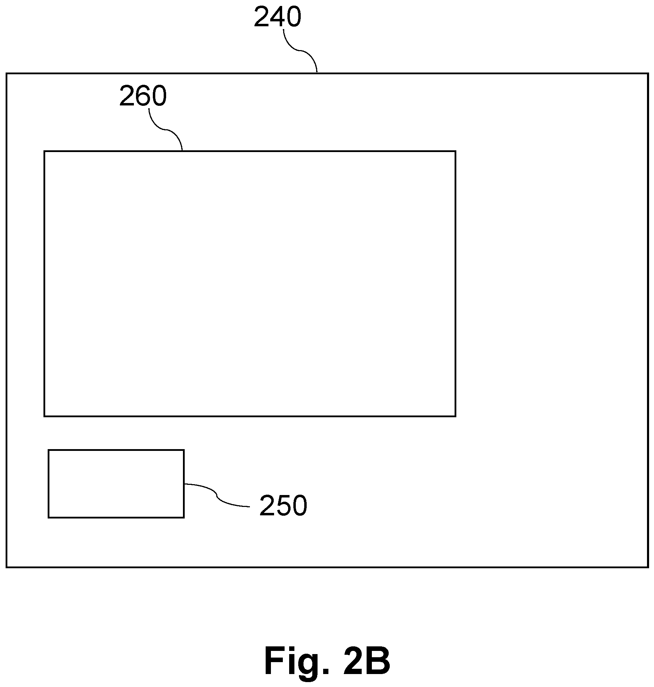

Fig. 2A shows a display 240 wherein the processor has selected the first window 250 as the prioritized window and the second window 260 as the non-prioritized window. The medical system settings have been altered to reduce the area of the display occupied by the non-prioritized window. Fig. 2B shows a display 240 wherein the processor has selected the second window 260 as the prioritized window and the first window 250 as the non-prioritized window. In this embodiment, the medical system settings are altered to reduce the area of the display occupied by the non-prioritized window.

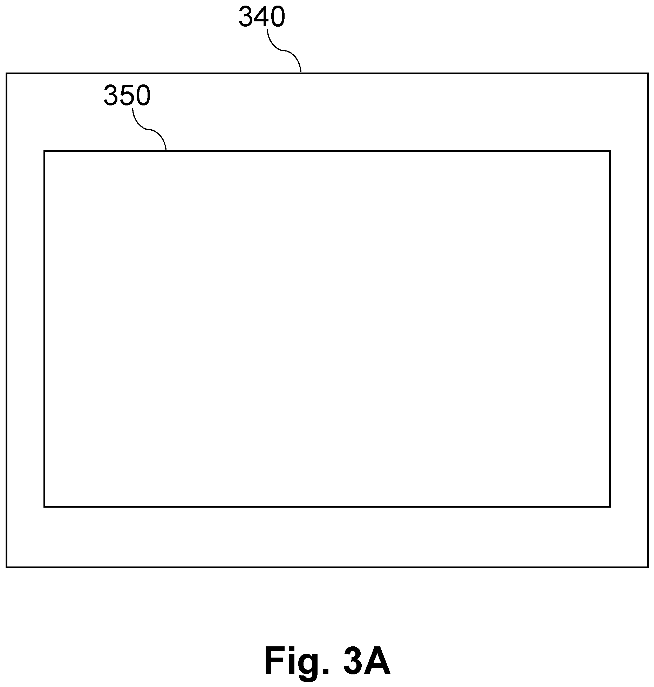

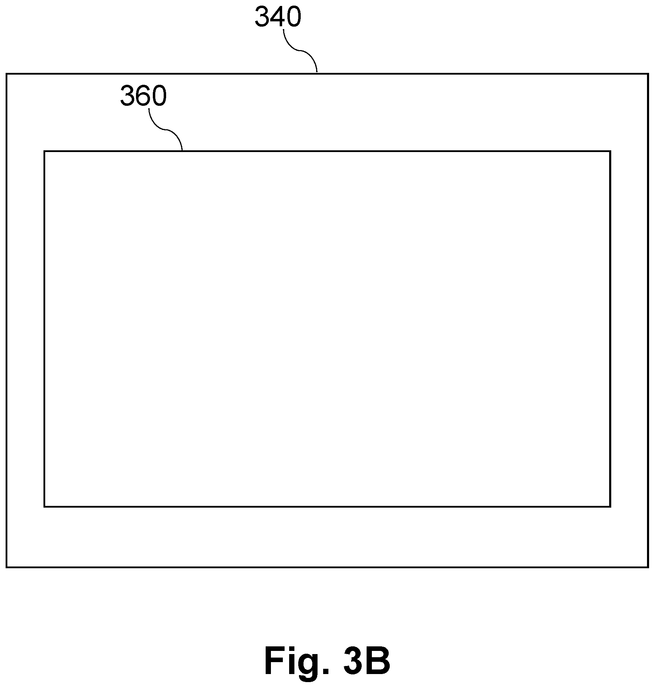

Fig. 3A shows a display 340 wherein the processor has selected the first window 350 as the prioritized window and the second window as the non-prioritized window. The medical system settings are altered to prevent the non-prioritized window from occupying any of the display area. Fig. 3B shows a display 340 wherein the processor has selected the second window 360 as the prioritized window and the first window as the non-prioritized window. Again, the medical system settings are altered to prevent the non-prioritized window from occupying any of the display area. Thus, for a value of a parameter at a time period, the display only shows either a first window or a second window.

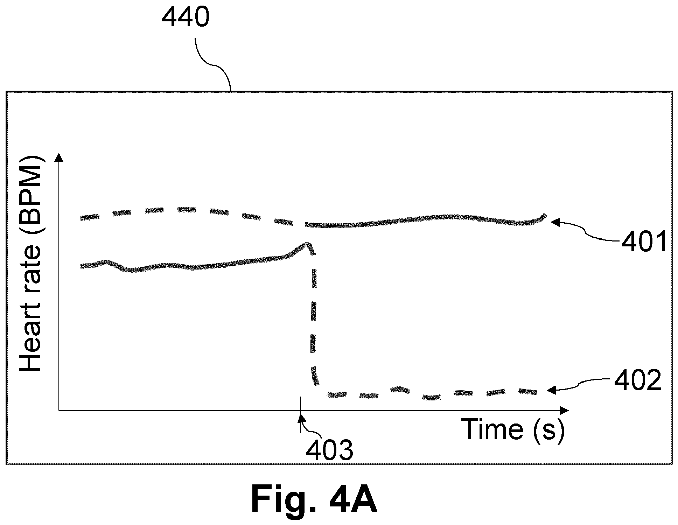

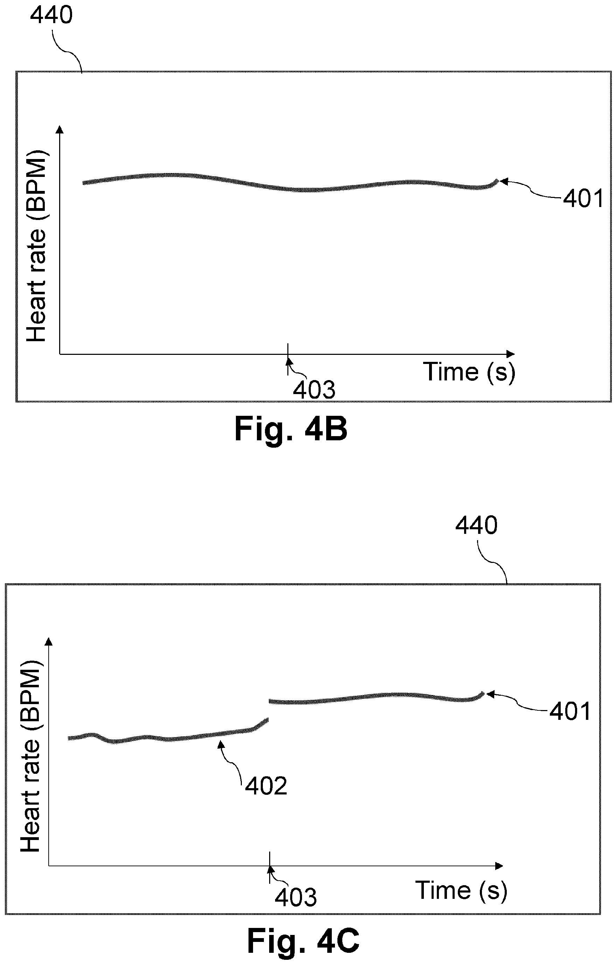

Figs 4A, 4B and 4C show embodiments wherein if there have been at least two prioritized methods of determining the value of the parameter within a time window, then the processor may be further configured to determine which option has a higher importance out of showing a prioritized value of the parameter at each time period within the time window and showing a single trend of the value of the parameter across the time window. A time window comprises at least two time periods. The option which is determined to have the higher importance is shown by the first window. A prioritized value of the parameter at each time period within the time window refers to the value of the parameter determined from the prioritized method at each time period within the time window. As this prioritized method is the most optimal method, as determined from the first set of predetermined criteria or user input, the prioritized value for each time period represents the most optimal value for each time period. A single trend of the value refers to the values determined from a single method. A single trend of the value of the parameter across the time window refers to the values of the parameter determined from a single method, which method was the prioritized method for at least one time period within the time window. In some embodiments, the single trend uses the values of the parameter from the prioritized method for the most recent time period within the time. Alternatively, the single trend uses the values of the parameter from the method which was the prioritized method for the most time periods of the time window. Alternatively, the single trend uses the values of the parameter from the method which was the prioritized method for at least one time period within the time window and has the highest accuracy out of all other prioritized methods. Showing a single trend shows how a parameter changes with time, without any kinks or disjointed regions where the prioritization of the method changes. This allows a more accurate display of the change of a parameter with time, which may provide a clinical advantage.

Fig. 4A shows a display 440 wherein the second window is the prioritized window for heart rate measurements. The display shows the value of heart rate on a graph, wherein the graph displays a single time window. The medical system settings are altered to reduce the area of the display 440 occupied by the non-prioritized method to zero. In this example, there are two sensors which measure heart rate, an SpOz sensor (shown as line 401) and an ECG sensor (shown as line 402). For the first time periods (that is, before time instance 403), the ECG is determined to the prioritized method. However, at the time instance 403, the ECG leads are unintentionally removed from the patient, and the system re-ranks and generates the SpOz method as the new prioritized method at this and future time periods. As Fig. 4A shows the second window, both values are shown at every time period. The prioritized method at each time period may be distinguished from the non-prioritized method in a variety of ways. In this embodiment, the prioritized method is shown using a solid line and the non-prioritized method is shown using a dashed line.

The importance of the options may be determined using a second set of predetermined criteria and/or user input. The second set of predetermined criteria may include one or more of the factors of the first set of predetermined criteria. The second set of predetermined criteria may include temporal characteristics of the value, and/or frequency domain characteristics of the value. The second set of predetermined criteria may also include the parameter being determined. For example, for heart rate the single trend is more important, whereas for mean arterial blood pressure the prioritized values are more important. The second set of predetermined criteria may also include the patient's current medical status. For example, for a cardiac patient, the prioritized value may be more important than the single trend, or if the patient is experiencing hemodynamic instability, a single trend is more important. Optionally, the patient's current medical status includes their treatment history or outlook and if a dose of a medication needs to be determined, the prioritized values are more important. If it needs to be established whether a patient is responding to a type of medication, the trend is more important. The patient's medical status may also include the development of the patient with time. For example, it may be beneficial to see how a patient is developing with time, and if so, the single trend may have a higher importance. The second set of predetermined criteria may also include HCP's workflow phase. For example, during handover to another HCP, if the patient is stable, showing a prioritized value of the parameter at each time period within the time window may be more important than showing a single trend of the value of the parameter across the time window. In another example, during handover to another HCP, if the patient is unstable, the need for showing a single trend of the value of the parameter across the time window may have higher importance. The determination of importance may also be based on manual input. For example, manual input may change the determination of importance. Manual input includes a user input, user interaction or any other manual input. A non-exhaustive list of examples includes but is not limited to input/interaction from a user via a touch screen, computer mouse, button, gesture control, keypad or voice control. The input may also come via a secondary device such as a mobile device or tablet.

Fig. 4B shows a display 440 wherein the first window is selected as the prioritized window and the medical system settings are altered to reduce the area of the display 440 occupied by the non-prioritized method to zero. The display shows the value of heart rate on a graph, wherein the graph represents a single time window. In this embodiment, there have been at least two prioritized methods of determining a value of heart within a time window, and showing a single trend of the value heart rate across a time window has a higher importance at the most recent time period. Based on this determination of higher importance, the first window shows the option which has the higher importance. Therefore, in the first window, the values of the parameter determined by the prioritized method at the most recent time period are shown for every time period within the time window. In other words, a single trend of heart rate is shown for the time window. The most recent prioritized method is the SpOz sensor, and so the values of heart rate from this sensor 401 are shown for the whole time window. This is despite the SpOz sensor not being the prioritized method of determining heart rate up to time instance 403. A trend line from a single method is shown across the whole time window, providing a more accurate representation of the changing of a parameter with time.

Fig. 4C shows a display 440 wherein the first window is selected as the prioritized window. The medical system settings are altered to reduce the area of the display 440 occupied by the non-prioritized method to zero. The display shows the value of heart rate on a graph, wherein the graph represents a single time window. In this example, there have been at least two prioritized methods of determining a value of a parameter within a time window, and showing the prioritized value of the parameter at every time period within a time window has a higher importance. In this case, in the first window the values of the parameter determined by the prioritized method at each time period within the time window are shown. The prioritized method for time periods up to time instance 403 is the ECG sensor, whereas after time instance 403 it is the SpOz sensor. Thus, for time periods up to time instance 403 the ECG 402 sensor is shown and in time periods after time instance 403 the SpOz sensor 401 is shown. This provides the most optimal value at each time period.

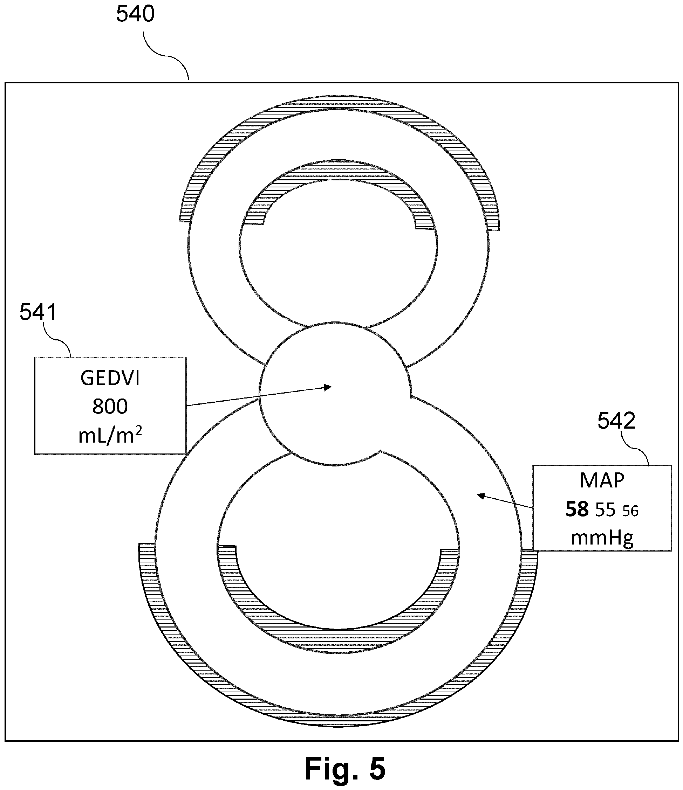

Fig. 5 shows a display 540 showing values of a parameter on a cardiopulmonary model. The model shows the values of GEDVI and MAP. The value of GEDVI is shown at a first region of the display 541 and the value of MAP is shown at a second region of the display 542. The first and second regions of the display may select the first or second windows as the prioritized window. If the first window is the prioritized window, the medical system settings may be adjusted to make second window either not shown, shown smaller, shown in a less prominent region of the display, shown more transparent, shown in a different style or any other method of distinguishing the non-prioritized window from the prioritized window. In this embodiment, the prioritized method determines a value of GEDVI of 800 mL/m². Here, the first window is selected for the value of GEDVI, and the medical system settings are adjusted to only show the prioritized value. For MAP, the second window is selected, and all values of MAP are shown. In the second window, the prioritized value is shown in the largest font, while the non-prioritized values are shown in smaller font. The ranking of the non-prioritized values of MAP is also shown by the decreasing size of the text. Here, 58 mmHg is the prioritized value, 55 mmHg the second ranked value and 56 mmHg the third ranked value. The cardiopulmonary model can show values of parameters graphically. For example, the thickness of a blood vessel may indicate the value of a parameter, for example MAP. The colors of the cardiopulmonary model can also indicate the value of the parameter, for example blood oxygen levels. The cardiopulmonary model in Fig. 5 only shows two parameters, but it is possible to show a plurality of parameters. Each parameter may prioritize a first window or a second window for any one time period, and one parameter may prioritize a second window whilst a different parameter is simultaneously prioritizing a first window.

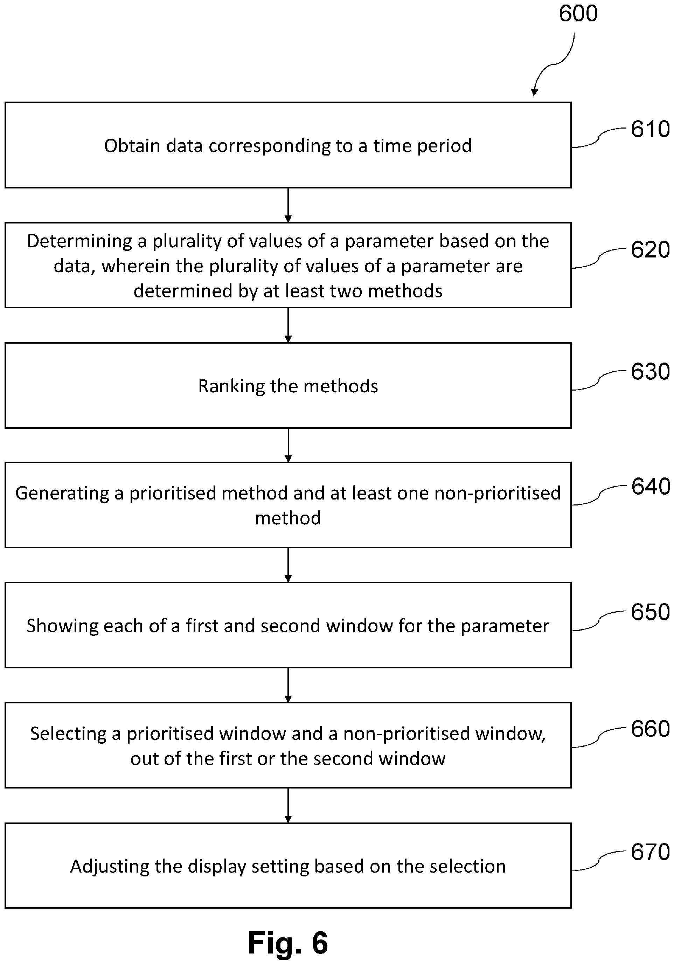

Fig. 6 shows a flow diagram of a method 600 of displaying health data using a medical system 100 according to aspects of the present disclosure. As illustrated, the method 600 includes a number of steps, but aspects of the method 600 may include additional steps before, after, or in between the steps. In some aspects, one or more of the steps may be omitted, performed in a different order, or performed concurrently.

Aspects of the method 600 may describe methods of providing HCPs with the most relevant information for the present clinical setting.

At step 610 the method 600 includes obtaining data by a sensor 110, the data corresponding to a time period. There may be various different types of sensor 110 obtaining data. The data may include data relating to various physiological parameters of a patient. There may be more than one sensor 110 obtaining data for the same physiological parameter.

At step 620 the method 600 includes determining a plurality of values of a parameter based on the data obtained by the sensor 110. The plurality of values of a parameter are determined by at least two methods. Methods may comprise determining data from different sensors 110 or from the same sensor 110 but using different algorithms. The sensor 110 may transmit the data to the processor 120 through wired or wireless means. The processor 120 then receives the data and determines one or more values of a parameter based on the data. The processor 120 may determine a plurality of values of a parameter based on data from a single sensor 110, optionally by using a plurality of algorithms to determine the values of a parameter. The processor 120 may determine the same number of values of a parameter as the number of sensors 110, optionally by only using one algorithm for each sensor's data. The processor 120 may determine a plurality of values of a parameter.

At step 630 the method 600 includes ranking the methods of determining the values of a parameter. The processor 120 may perform this ranking, and the ranking may be based on a variety of methods, including but not limited to a predefined ranking of sensors 110, the accuracy of the value of the parameter, the delay time between the obtaining of data by a sensor 110 and the determining of the value of the parameter by the processor 120, the computational cost needed to determine the value of the parameter, the battery level of the sensor 110, the memory requirements of the value of the parameter, the favored sensor 110 in the current care setting, or predictive algorithms. Details of how the ranking may be performed based on these methods have been introduced above. The ranking may be performed based on any combination of the methods used in the above list. For example, the ranking may be performed based on the accuracy of the value of the parameter and the delay time of the sensor 110.

At step 640, the method 600 includes generating a prioritized method of determining the value of the parameter and at least one non-prioritized method of determining a value of the parameter based on the ranking. The prioritized value of the parameter may be the top ranked method, and the other ranked values may be the non-prioritized methods.

At step 650, the display shows each of a first window and a second window. Each means the display may only show with a first window or a second window for a parameter at any time period. The display may show both the first and second window for a parameter at a time period simultaneously, with one of the first or second window shown in a more dominant, prominent or primary location on the screen.

In the first window, values of the parameter determined by the non-prioritized methods for the time period are not shown. In the first window, the only value of the parameter for the time period shown is the value determined by the prioritized method, and no value determined by other methods is shown. In the second window the values of the parameter determined by the non-prioritized methods for the time period are shown. The non-prioritized methods may include all of the methods of the parameter. In other words, all of the values of the parameter are shown. The prioritized value of the parameter may also be shown in the second view.

At step 660, the method 600 includes selecting a prioritized window and a non-prioritized window, out of the first or the second window. The method may select the prioritized and non-prioritized window based on predetermined criteria and or user interaction. The predetermined criteria may include one or more of the following factors: a value of the parameter reaching a threshold value, the use of a new sensor 110, the removal of a sensor 110, the battery life of a sensor 110, the previous time each window has been shown, a reduction in the size of the display 140, a patient's medical status, a healthcare professionals preferences or a healthcare professional's shift data and the difference between the values of a parameter. Details of the predetermined criteria have been introduced above. A non-exhaustive list of examples of user interaction includes input from a user via a touch screen, computer mouse, button, keypad, gesture control, or voice control. The manual input may also be further configured to override the automatic selection of the window to be shown by the display 140.

At step 670, the method 600 includes adjusts a setting of the medical system based on the selection of the prioritized and non-prioritized window. The medical system settings may be adjusted in various ways to enable the prioritized window to be more dominant than the non-prioritized window. For example, the prioritized window may take up a greater proportion of the display area relative to the non-prioritized window. Optionally, the non-prioritized window may not take up any of the display's area. Alternatively, the non-prioritized window may be more transparent than the prioritized window, and/or in lighter or less visible colors than the prioritized window. The prioritized and non-prioritized windows may occupy the same region of the display, or overlapping regions of the display, or different regions of the display.

The value of a parameter may be a physiological parameter corresponding to the patient. The physiological parameter may include but are not limited to heart rate, blood pressure, respiration rate (RR), body temperature, blood oxygen (SpO2), global end-diastolic volume (GEDVI), systemic vascular resistance index (SVRI), continuous cardiac index (CCI), stroke volume index (SVI), extravascular lung water index (ELWI), central venous pressure (CVP), systolic blood pressure (SBP), mean arterial pressure (MAP).

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 1 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A medical system (100) comprising:
at least one sensor (110), configured to obtain data corresponding to a time period;
a processor (120) configured to:
determine a plurality of values of a parameter based on the data corresponding to the time period, wherein the plurality of values of a parameter are determined by at least two methods,
rank the methods, and
generate a prioritized method for the time period and at least one non-prioritized method for the time period based on the ranking, and
a display (140) configured to show each of a first window and a second window for a parameter,
wherein in the first window the values of the parameter determined by the non-prioritized methods for the time period are not shown,
wherein in the second window at least one of the values of the parameter determined by the non-prioritized methods for the time period are shown, and
wherein the processor further comprises a selector (130) configured to select a prioritized window and a non-prioritized window, out of the first or the second window, wherein the selection adjusts a setting of the medical system.

2. The medical system of claim 1 wherein the medical system setting comprises the display area, and the medical system setting is adjusted to not show the non-prioritized window on the display area.

3. The medical system of any preceding claim, wherein the methods comprise determining a plurality of values of a parameter from one or more of different sensors and different algorithms.

4. The medical system of any preceding claim, wherein the selector is further configured to automatically select the prioritized window and the non-prioritized window, out of the first or the second window, based on a first set of predetermined criteria.

5. The medical system of claim 4, wherein the first set of predetermined criteria includes the difference between the values of a parameter.

6. The medical system of claim 4, wherein the first set of predetermined criteria includes one or more of the following factors: a value of the parameter reaching a threshold value, the use of a new sensor, the removal of a sensor, the battery life of a sensor, the previous time each window has been shown, a reduction in the area of the display available to show a first or second window, a patient's medical status, a prediction of the patient's future medical status, a healthcare professional's preferences, and a healthcare professional's workflow phase.

7. The medical system of any preceding claim, wherein the selector is further configured to select the prioritized window and the non-prioritized window, out of the first or the second window, based on a user interaction with the system.

8. The medical system of claims 4-6, wherein the selector is further configured to select the prioritized window and the non-prioritized window, out of the first or the second window, based on a user interaction with the system and wherein the user interaction with the system overrides the automatic selection.

9. The medical system of any preceding claim, wherein after generating an initial prioritized method and at least one non-prioritized method, the processor only performs a further rank of the methods after a plurality of time periods.

10. The medical system of any preceding claim, wherein the ranking is performed using one of following means: a predefined ranking of sensors, the accuracy of the value of the parameter, the reliability of the value of the parameter, the delay time between the patient's physiology and the determining of the value of the parameter by the processor, the computational cost needed to determine the value of the parameter, the battery level of the sensor, the memory requirements of the value of the parameter, a custom ranking, the favored sensor in the previous, current, or future care setting, or predictive algorithms.

11. The medical system of any preceding claim, wherein if there have been at least two prioritized methods of determining the value of the parameter within a time window, wherein a time window comprises at least two time periods, then:
the processor is further configured to determine, using a second set of predetermined criteria and/or user input, which option has the higher importance out of
showing a prioritized value of the parameter at each time period within the time window, wherein the prioritized value of the parameter at each time period within the time window refers to the value of the parameter determined from the prioritized method at each time period within the time window; and
showing a single trend of the value of the parameter across the time window, wherein the single trend of the value of the parameter across the time window refers to the values of the parameter determined from a single method, which method was the prioritized method for at least one time period within the time window, and
the first window shows the option with the higher importance.

12. The medical system of claim 11, wherein the second set of predetermined criteria includes one or more of the following factors: the parameter being determined, the patient's current medical status, the patient's historic medical status, a prediction of the patient's future medical status, the percentage of the time window that a method was the prioritized method, temporal characteristics of the value, the difference between the values of the parameter, a value of the parameter reaching a threshold value, the use of a new sensor, the removal of a sensor, the battery life of a sensor, the previous time each window has been shown, a reduction in the area of the display available to show a first or second window, a patient's medical status, a prediction of the patient's future medical status, a healthcare professional's preferences, and a health care professional's workflow phase.

13. The medical system of any preceding claim, wherein if the first window is selected as the prioritized window, the non-prioritized methods are put into a sleep mode.

14. The medical system of any preceding claim:
wherein the processor is further configured to determine uncertainty data for the plurality of values of a parameter; and
wherein in the first and/or second window the uncertainty data is shown.

15. A method of displaying health data using a medical system,
the medical system comprising:
at least one sensor;
a display; and
a processor, further comprising a selector;
the method comprising:
obtaining, by the at least one sensor, data corresponding to a time period;
determining, by the processor, a plurality of values of a parameter based on the data corresponding to a time period, wherein the plurality of values of a parameter are determined by at least two methods;
ranking, by the processor, the methods;
generating, by the processor, a prioritized method for the time period and at least one non-prioritized method for the time period based on the ranking;
showing, by the display, each of a first window or a second window,
wherein in the first window the values of the parameter determined by the non-prioritized methods for the time period are not shown, and
wherein in the second window at least one of the values of the parameter determined by the non-prioritized methods for the time period are shown; and
selecting, by the selector, a prioritized window and a non-prioritized window, out of the first or the second window, wherein the selection adjusts a setting of the medical system.
